# EUROPEAN PATENT APPLICATION

(11) **EP 4 516 334 A1**
(43) Date of publication of application: **05.03.2025**
(21) Application number: 23195150.0
(22) Date of filing: 04.09.2023
(51) Int. Cl.: A61M 5/00

(54) **MEDICAL CONTAINER NEST WITH REDUCED WARPAGE AND RESIDUAL STRESS**

(71) Applicant: Becton, Dickinson and Company, Franklin Lakes, NJ 07417-1880 (US)
(72) Inventor: PAULET, Mathilde, 38100 Grenoble (FR); RIVE, Adrien, 38000 GRENOBLE (FR); VONIEZ, Jimmy, 38000 GRENOBLE (FR); REMPFER, Simon, 38260 St Hilaire de la Cote (FR)
(74) Representative: Germain Maureau

(57) **Abstract**

A nest configured to support medical containers is provided. The nest includes a support plate comprising a first surface, a second surface, and a peripheral edge between the first surface and the second surface, with the support plate configured as a frame-shaped plate having a hollow interior area. The nest also includes a plurality of chimneys extending outwardly and away from the first surface that define receptacles configured to receive medical containers therein. The chimneys include an interior chimney group positioned in the hollowed interior area of the frame-shaped plate and a perimeter chimney group surrounding the interior chimney group, with each of the perimeter chimneys at least partially joined to an inner edge of the frame-shaped plate at the bottom end thereof. The nest further includes reinforcing ribs extending between the plurality of chimneys that connect each respective chimney to a plurality of adjacent chimneys.

## Description

### BACKGROUND OF THE INVENTION

### Field of the Invention

The present disclosure relates generally to holding devices for holding medical containers, such as cartridges, barrels, prefilled-syringes, stoppers, and/or pre-fillable syringes, in an upright position and, in particular, to medical container nests including a plurality of chimneys configured to retain the medical containers therein, with ribs extending between at least some of the plurality of chimneys to increase a rigidity of the support plate.

### Description of Related Art

Medical containers, such as cartridges, barrels, prefilled-syringes, stoppers, and/or pre-fillable syringes, often need to be transported from one site to another site during or after manufacturing. For example, medical containers may be manufactured at a first site and then transported to a second site, where the medical containers are filled with a medical fluid. As used herein, a "medical fluid" can refer to a medication or another therapeutic agent used for treatment of chronic or acute conditions, as are known in the art. Exemplary therapeutic agents can include, for example, drugs, chemicals, biological, or biochemical substances that, when delivered in a therapeutically effective amount to the patient, achieve a desired therapeutic effect. In other examples, the medical containers can be manufactured and filled at a same first site and then transported to a second site for storage.

During transport, the medical containers can be held by a holding device, such as a tray or "nest." A nest can be a plate-shaped tray configured to support multiple medical containers in an upright orientation. Nests can comprise multiple "chimneys" aligned according to predetermined rows or a predetermined pattern, with each chimney defining a receptacle or opening configured to receive one medical container. When inserted into and through the receptacle, the medical container is held in the upright orientation in a direction substantially orthogonal to the nest. The nest is usually placed inside a box-shaped tub with an open top, which can be sealed by a sealing cover. Removal of the medical containers from this tub can require peeling off the sealing cover, removing the nest holding the medical containers from the tub, and removing the medical containers from the nest by axially sliding the medical containers relative to the nest.

As known in the art, nests as described above are typically formed via an injection molding process, in which the nest is formed by injecting a fluid polymer material between a first tool and a second tool in a single process - i.e., the support plate and chimneys of the nest are integrally formed from a same material. For example, the nests of the present disclosure can comprise a thermoplastic material, preferably polypropylene, or polyester, polycarbonate, polyethylene, polyethylene terephthalate, acrylonitrile butadiene styrene, or other injection moldable or formable resin materials, as are known in the art.

A recognized drawback to the molding of nests as described above is the tendency for the nest to warp over time. That is, due to the molding material(s) used and the structure of the nest (i.e., a flat support plate of larger size, with a plurality of chimneys extending out therefrom), the nest is prone to experiencing a "natural" warpage or deformation that occurs due to residual stress present inside the nest component (due to the injection molding process) that is released over time. As a result of the warpage/deformation of the nest, medical containers supported by the deformed nest may not be retained in the upright orientation, which may lead to some inaccuracy during an automated filling process and/or during a stoppering process. Deformation of the nest may also cause problems during conveying of the filled nest to different locations.

For these reasons, there is a need for nest designs that are less sensitive to natural warpage (and deformation) that might result from the molding process of the nest. The nest designs would not only be less sensitive to natural warpage, but would also provide increased stiffness in order to reduce deflection of the nest or tray during transport and filling. Avoiding warpage and deformation can improve accuracy of the filling process by avoiding misalignment between the medical containers contained by the tray or nest and the automated filling machines.

### SUMMARY OF THE INVENTION

According to an aspect of the disclosure, a nest configured to support medical containers is provided. The nest includes a support plate comprising a first surface, a second surface, and a peripheral edge between the first surface and the second surface, with the support plate configured as a frame-shaped plate having a hollow interior area. The nest also includes a plurality of chimneys extending outwardly and away from the first surface, each of the plurality of chimneys defining a receptacle configured to receive a medical container therein, with each of the plurality of chimneys having a top end defining an upper opening and a bottom end defining a lower opening, and wherein the plurality of chimneys includes an interior chimney group comprising a plurality of interior chimneys, the interior chimney group positioned in the hollowed interior area of the frame-shaped plate, and a perimeter chimney group surrounding the interior chimney group, the perimeter chimney group comprising a plurality of perimeter chimneys, with each of the plurality of perimeter chimneys at least partially joined to an inner edge of the frame-shaped plate at the bottom end thereof. The nest further includes reinforcing ribs extending between the plurality of chimneys, the reinforcing ribs connecting each respective chimney to a plurality of adjacent chimneys.

In accordance with an aspect of the disclosure, the reinforcing ribs are orthogonal to the support plate and extend up from a bottom end of a respective chimney to a height that is a quarter or greater than a height of the chimney.

In accordance with an aspect of the disclosure, the height of each of the reinforcing ribs is equal to the height of each of the plurality of chimneys, such that the reinforcing rib extends from the bottom end to the top end of each chimney.

In accordance with an aspect of the disclosure, an arrangement of six reinforcing ribs connects each of the plurality of interior chimneys to six adjacent chimneys in a hexagonal arrangement.

In accordance with an aspect of the disclosure, an arrangement of four reinforcing ribs connects each of the plurality of interior chimneys to four adjacent chimneys in a cross-shaped arrangement.

In accordance with an aspect of the disclosure, the nest further includes a plurality of plate sections positioned in the hollow interior area and in a planar arrangement with the support plate, each of the plate sections positioned between a grouping of chimneys of the plurality of interior chimneys, wherein a bottom edge of each of the reinforcing ribs joining a respective grouping of chimneys is joined with a respective plate section.

In accordance with an aspect of the disclosure, the plurality of chimneys are configured to receive syringes therein from 0.5 to 50 mL in volume.

In accordance with an aspect of the disclosure, each of the plurality of chimneys comprises a tubular wall comprising a cylindrical inner surface having a diameter substantially the same as the diameter of the upper and lower openings of the chimney and a cylindrical outer surface opposite the inner surface.

In accordance with an aspect of the disclosure, the support plate, the plurality of chimneys, and the reinforcing ribs are integrally formed and are made from a thermoplastic polymer.

In accordance with an aspect of the disclosure, when fully loaded with the medical containers, deflection of the support plate is less than 1.0 mm in a vertical direction.

According to another aspect of the disclosure, a nest configured to support medical containers is provided. The nest includes a support plate comprising a first surface, a second surface, and a peripheral edge between the first surface and the second surface, and a plurality of chimneys extending outwardly and away from the first surface, with each of the plurality of chimneys defining a receptacle configured to receive a medical container therein, and with each of the plurality of chimneys having a top end defining an upper opening and a bottom end defining a lower opening. Reinforcing ribs extend between at least some of the plurality of chimneys, the reinforcing ribs connecting a respective chimney to a one or more adjacent chimneys. The reinforcing ribs are orthogonal to the support plate and extend up from the bottom end of a respective chimney to a height that is one half or greater of a height of the chimney.

In accordance with an aspect of the disclosure, the height of each of the reinforcing ribs is equal to the height of each of the plurality of chimneys, such that the reinforcing rib extends from the bottom end to the top end of each chimney.

In accordance with an aspect of the disclosure, the plurality of chimneys includes an interior chimney group comprising a plurality of interior chimneys and a perimeter chimney group surrounding the interior chimney group, the perimeter chimney group comprising a plurality of perimeter chimneys.

In accordance with an aspect of the disclosure, the support plate comprises a frame-shaped plate having a hollow interior area, with the interior chimney group positioned in the hollowed interior area of the frame-shaped plate and with each of the plurality of perimeter chimneys at least partially joined to an inner edge of the frame-shaped plate at the bottom end of the chimney.

In accordance with an aspect of the disclosure, the support plate comprises a pair of cutouts extending inwardly from the peripheral edge thereof, with a first cutout formed on a first transverse side of the support plate and a second cutout formed on a second transverse side of the support plate, and wherein the reinforcing ribs comprise a first arrangement of reinforcing ribs connecting each of a plurality of chimneys in a line of chimneys arranged between the first cutout and the second cutout and a second arrangement of reinforcing ribs connecting each of the plurality of perimeter chimneys to an adjacent perimeter chimney.

In accordance with an aspect of the disclosure, an arrangement of reinforcing ribs connects each of the plurality of interior chimneys to chimneys adjacent thereto, the arrangement of reinforcing ribs comprising an arrangement of six reinforcing ribs connecting each of the plurality of interior chimneys to six adjacent chimneys or an arrangement of four reinforcing ribs connecting each of the plurality of interior chimneys to four adjacent chimneys in a cross-shaped arrangement.

### BRIEF DESCRIPTION OF THE DRAWINGS

FIG. 1 is a perspective view of a nest configured to support medical containers, according to an aspect of the present disclosure;
FIG. 2 is a top view of the nest of FIG. 1;
FIG. 3 is a cross-sectional view of the nest of FIG. 2;
FIG. 4 is a perspective view of a nest configured to support medical containers, according to another aspect of the present disclosure;
FIG. 5 is a top view of the nest of FIG. 4;
FIG. 6 is a perspective view of a nest configured to support medical containers, according to another aspect of the present disclosure;
FIG. 7 is a top view of a nest configured to support medical containers, according to another aspect of the present disclosure;
FIG. 8 is a cross-sectional view of the nest of FIG. 7; and
FIG. 9 is a perspective view of a nest configured to support medical containers, according to another aspect of the present disclosure.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", "transverse", and derivatives thereof shall relate to the invention as it is oriented in the drawing figures. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

With reference to the figures, the present disclosure is directed to a holding device or "nest" 10 comprising chimneys or receptacles for retaining multiple medical containers, such as multiple cartridges, barrels, stoppers, prefilled-syringes, and/or pre-fillable syringes, in an upright position. In particular, the nest 10 can be configured to concurrently support multiple medical containers in the upright position during packaging, storing, transporting, and/or handling of the multiple medical containers with an automated filling machine.

A medical container, such as a syringe, is generally an elongate structure that comprises a hollow elongated barrel defining a reservoir for containing the medical fluid, a stopper located inside the barrel, and a plunger rod to move the stopper through the barrel to expel the medical fluid through a tip, fluid port, nozzle, or needle cannula at an end of the barrel. The reservoirs of the medical containers held by the nest 10 of the present disclosure can contain from 0.5 to 50 mL, or preferably from about 1 mL to 10 mL, of the medical fluid. The medical containers can also include a flange at an end of the barrel, which can provide a surface for positioning a user's fingers (e.g., the index and the middle fingers), while the plunger rod is activated with the thumb. The nest 10 can be configured to retain 160 or fewer medical containers, and preferably 100 or fewer medical containers.

According to aspects of the disclosure, the nest 10 can include features that limit (natural) warpage of the nest 10 that may result from manufacturing thereof and/or that increase the stiffness of the nest 10. In particular, the nest 10 can be configured to limit warpage during manufacturing (i.e., molding) of the nest 10 and/or resist deformation of the nest 10 during conveying thereof, after medical containers held thereby have been filled.

According to aspects of the disclosure, the nest 10 can be designed to reduce residual stress in the nest 10 that might be present resulting from molding of the nest 10.

According to additional aspects of the disclosure, the nest 10 can be configured to resist deformation after medical containers held thereby are fully loaded. As one example, for a nest 10 configured to include 100 chimneys loaded with filled 1-3 mL medical containers, the nest 10 may deflect by about 0.57 mm or less (depending on an exact arrangement of externally located supports that hold the nest), as determined by finite element analysis (FEA). By contrast, a conventional nest including 100 chimneys, but with conventional reinforcing ribs, was found by FEA to deflect by approximately 85% more as compared to the nest(s) of the present disclosure. In other embodiments, the nest 10 can be configured to include forty-two (42) chimneys loaded with filled 10 mL medical containers, with the nest again exhibiting reduced deflection as compared to a nest including forty-two chimneys but with conventional reinforcing ribs.

As previously described, the nest 10 of the present disclosure is configured to be disposed in a container, such as a tub. For example, the nest 10 of the present disclosure can be disposed across the open top of a tub to cover an interior of the tub. The medical containers can be inserted through chimneys of the nest 10, such that distal portions of the medical containers (i.e., portions of the medical containers that are farthest away from the portion of the device or container grasped or manipulated by a user) are in the interior of the tub and a sidewall of the medical container is supported by the chimney of the nest 10.

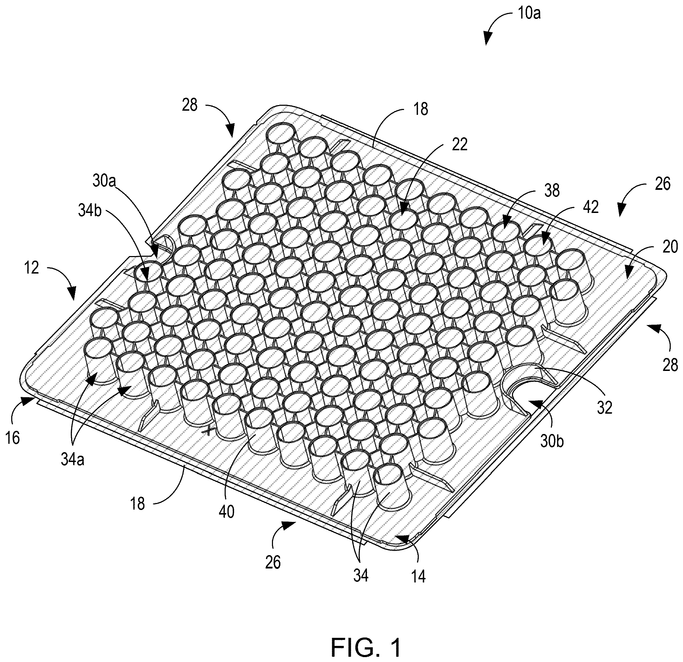

With reference now to the figures, FIGS. 1-3 illustrate an exemplary nest 10a configured to support medical containers, according to an aspect of the present disclosure. The nest 10a comprises a support plate 12 having a first or upper surface 14, a second or lower surface 16, and a peripheral edge 18 extending about the support plate 12 between the upper surface 14 and the lower surface 16. The support plate 12 is structured as a frame-shaped plate having an exterior area 20 and a hollow interior area 22. The exterior area 20 is defined between the peripheral edge 18 and an inner edge 24 and surrounds the hollow interior area 22.

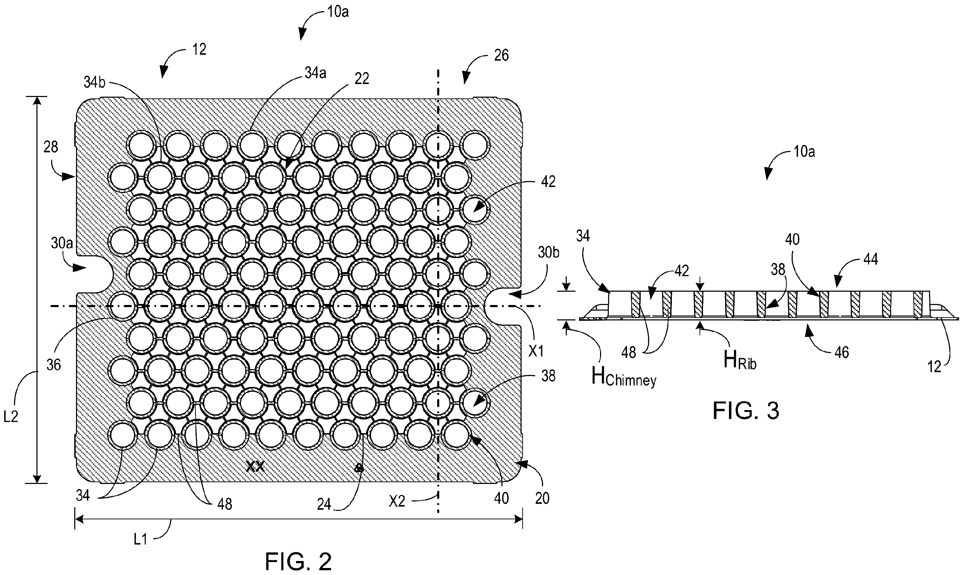

In some examples, the perimeter of support plate 12 is substantially rectangular in shape - having longitudinal sides 26 and transverse sides 28. As used herein, the "longitudinal sides" refer to sides 26 of the support plate 12 having a length L1 (shown in FIG. 2) and the "transverse sides" refer to sides 28 of the support plate 12 having a length L2. In some embodiments, the length L1 of longitudinal sides 26 is longer than the length L2 of transverse sides 28. In other embodiments, the support plate 12 may be square, in which case the longitudinal sides 26 and the transverse sides 28 are the same length (i.e., L1 is equal to L2). As shown in FIG. 2, the substantially rectangular support plate 12 defines a first axis X1 (also referred to as a longitudinal, horizontal, or x axis) extending parallel to the longitudinal sides 26 of the support plate 12 and a second axis X2 (also referred to as a transverse, latitudinal, vertical, or y axis) extending parallel to the transverse sides 28 of the support plate 12. Dimensions of the support plate 12 will be determined by those skilled in the art based on the number of medical containers that can be supported by the nest 10a, dimensions of the medical containers, and/or dimensions of the tub or container to which the nest 10a is mounted.

The support plate 12 can also include cutouts 30a, 30b extending inwardly from other portions of the peripheral edge 18. For example, as shown in FIGS. 1 and 2, the support plate 12 includes a first cutout 30a on one of the transverse sides 28 of the support plate 12 and a second cutout 30b on a second transverse side 28 of the support plate 12. The first and second cutouts 30a, 30b can be aligned along the longitudinal or first axis X1 of the support plate 12. In some examples, the cutouts 30a, 30b are a u-shaped gap or opening extending inwardly from the peripheral edge 18 of the support plate 12. The nest 10a can also include a curved wall 32 extending from the upper surface 14 and/or the lower surface 16 of the support plate 12 and about at least a portion of the first cutout 30a and/or the second cutout 30b.

The nest 10a further comprises tubular walls, members, or ridges - referred to hereafter as "chimneys 34" - that protrude outward and upward from a plane defined by the upper surface 14 of the support plate 12. Each chimney 34 can include a cylindrical wall 36 comprising a cylindrical inner surface 38 having an inner diameter and a cylindrical outer surface 40 opposite the inner surface 38 and having an outer diameter. The cylindrical inner surface 38 of each chimney 34 defines a receptacle 42 configured to receive a medical container therein. The receptacle 42 includes a top opening 44 at a top end of the chimney 34 (i.e., a top edge of cylindrical wall 36) and a bottom opening 46 at a bottom end of the chimney 34 (i.e., a bottom edge of cylindrical wall 36).

The receptacles 42 are sized to support medical containers in an upright position. For example, the receptacles 42 can be wide enough so that a sidewall or barrel of the medical container passes through the receptacles 42, while preventing a flange or another protruding portion of the medical container from passing through the receptacles 42. Accordingly, when the medical container is inserted through one of the receptacles 42, the flange of the medical container may rest against the upper edge of the chimney 34, thereby holding the medical container in the upright position. The dimensions of the receptacles 42 are generally dependent upon a size of the medical container intended to be used with the nest 10a.

The chimneys 34 provided on nest 10a may be generally characterized as including perimeter chimneys 34a and interior chimneys 34b. The perimeter chimneys 34a are those chimneys that form a perimeter about the entire arrangement of chimneys 34, while the interior chimneys 34b are surrounded by the perimeter chimneys 34a. According to aspects of the disclosure, the perimeter chimneys 34a may be directly joined to (i.e., formed integrally with) the support plate 12, with a portion of the bottom end of each perimeter chimney 34a joined to the support plate 12. That is, each of perimeter chimneys 34a has a bottom end that is partially joined to the frame-shaped support plate 12 along the inner edge 24 thereof. Conversely, the interior chimneys 34b are positioned within the hollow interior area 22 of the frame-shaped support plate 12 and are not directly joined to the support plate 12, but instead are only joined to perimeter chimneys 34a via reinforcing ribs, as explained in detail below.

As previously described, the nest 10a includes structures for increasing stiffness of the support plate 12 and for resisting deflection or deformation when the nest 10a is fully filled with medical containers. In particular, the nest 10a includes a plurality of radial reinforcing ribs 48 that connect adjacent chimneys 34 one to each other for rigidifying the nest 10a. According to embodiments, each of the ribs 48 is structured as a planar, wall-shaped member that is oriented vertically and orthogonal to the support plate 12. Each rib 48 extends between an adjacent pair of chimneys 34, with the rib 48 being formed integrally with the chimneys 34 as part of a single nest structure. For an embodiment where the chimneys 34 are provided in a hexagonal arrangement, as shown in FIGS. 1 and 2, each interior chimney 34b will include six (6) ribs 48 extending radially outward therefrom to six adjacent chimneys 34 arranged hexagonally thereabout, while each perimeter chimney 34a will include at least two (2) ribs 48 extending radially outward therefrom to two adjacent chimneys 34.

According to an aspect of the disclosure, the ribs 48 have a height that adds a desired rigidity to the nest 10a, so as make the nest 10a more resistant to deflection or deformation when the nest 10a is fully filled with medical containers. The ribs 48 are thus constructed to have a height, H_{Rib}, that is at least a quarter of the height of the chimneys, H_{Chimney}, with a bottom edge of the ribs 48 being generally aligned with the bottom end of chimneys 34 and extending upwardly therefrom. According to some embodiments, each of ribs 48 may be constructed to have a height, H_{Rib}, that is equal to the height of the chimneys, H_{Chimney}, such that the ribs 48 extend a full length of the chimneys 34, between the bottom end and the top end thereof.

With the nest 10a structured as described above, the performance and longevity of the nest 10a can be improved as compared to previous nest designs. First, the increased height of the reinforcing ribs 48 formed between chimneys - with the ribs 48 having a height that is at least a quarter the height of the chimneys 34 - improves the ability of the nest 10a to resist deflection or deformation when the nest 10a is fully filled with medical containers. Second, by forming the support plate 12 as a frame-shaped plate having a hollow interior area 22, a portion of the nest 10a is removed that may contribute to unpredictable curvature and/or deflection (i.e., "flatness issues") after molding and/or after sterilization, even in nest designs incorporating reinforcing ribs. That is, it is recognized that an interior area 22 of the support plate 12 can cause part of the flatness issues due to differential shrinkage rates of that region of the support plate 12 compared to the chimneys 34, thereby leading to residual constraints in the nest 10a after the molding step. Removal of the interior area 22 of the support plate 12 by structuring the support plate as a frame-shaped plate can eliminate this contributor to flatness issues without negatively impacting the rigidity of the support plate 12 - as the interior area 22 of the support plate 12 contributes very minimally to the plate rigidity, as confirmed by FEA.

According to aspects of the disclosure, configuring of the nest 10a to include a frame-shaped support plate 12 with a hollow interior area 22 - as opposed to a continuous/solid support plate 12 - functions to eliminate some natural warpage of the nest that is caused by to residual constraints in the nest 10a after the molding step. According to FEA, the warpage of the nest 10a (and support plate 12 thereof) can be reduced by including hollow interior area 22 in support plate 12.

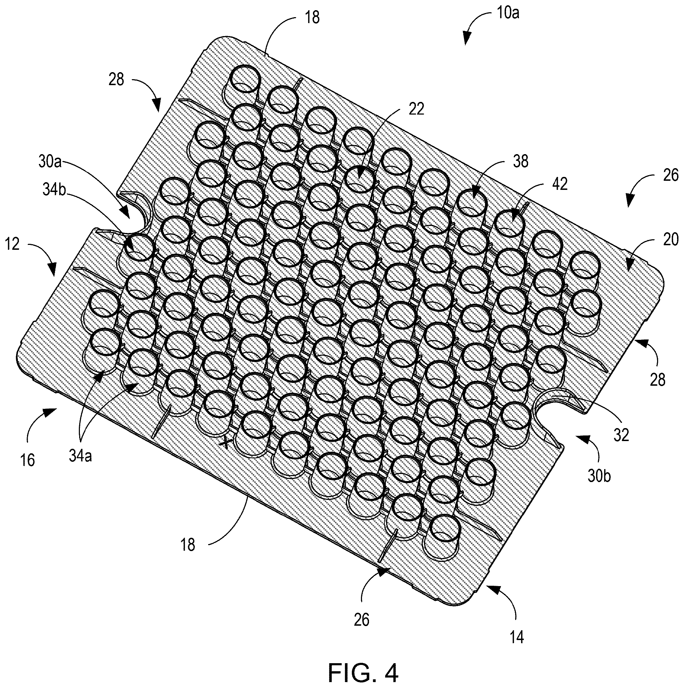

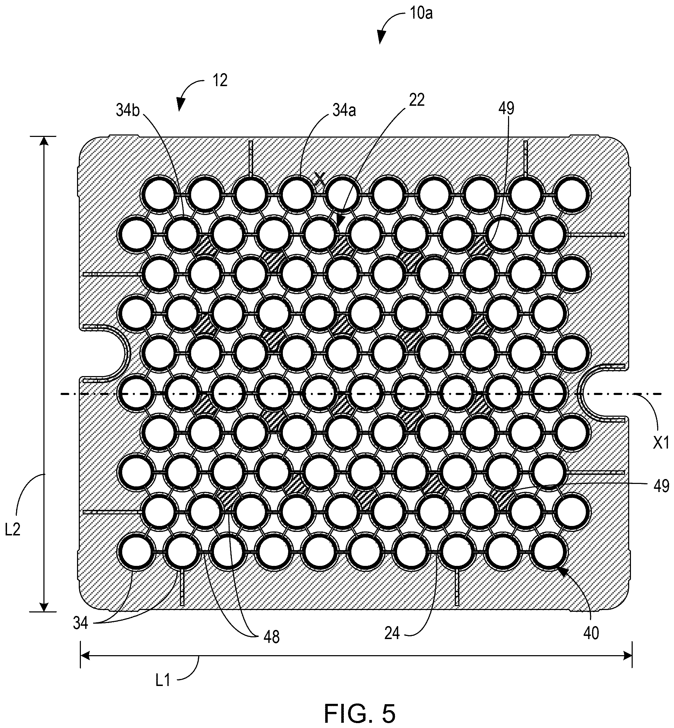

In some embodiments, the nest 10a may be modified to include a plurality of plate sections 49 positioned in the hollow interior area thereof, as shown in FIGS. 4 and 5. That is, a number of plate sections 49 (FIG. 5) may be provided between select groupings of interior chimneys 34b. The plate sections 49 are similar to other portions of the support plate 12 in thickness and construction, and the plate sections 49 are in a planar arrangement with the support plate 12. The plate sections 49 are joined to respective reinforcing ribs 48 and interior chimneys 34b for each grouping of chimneys 34b with which they are associated, with a bottom edge of each of the reinforcing ribs 48 extending between a respective grouping of chimneys 34b being joined with a respective plate section 49. The plate sections 49 may function to add stiffness/rigidity to the nest 10a to resist deflection or deformation thereof, while still configuring the nest 10a to be less sensitive to natural warpage by reducing residual stress therein resulting from a molding process.

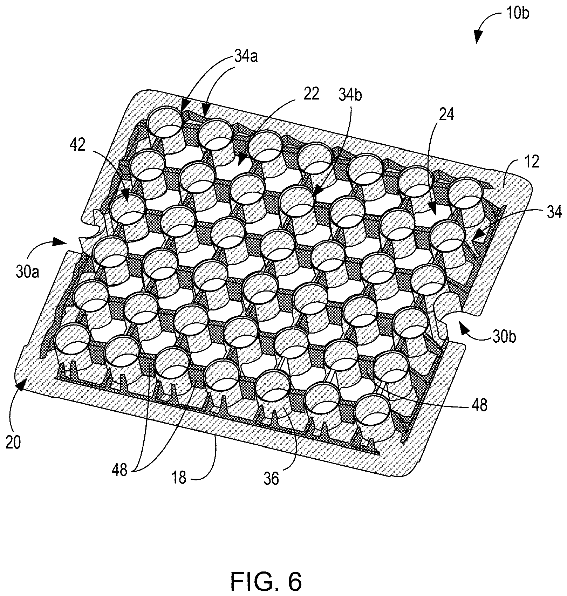

FIG. 6 illustrates another example of a nest 10b including optimized reinforcing ribs 48. The nest 10b is configured to include forty-two (42) chimneys 34 that accommodate 10 mL medical containers, with the chimneys 34 arranged in a plurality of aligned rows and columns.

The nest 10b is constructed similarly to the nest 10 previously shown and described in FIGS. 1-3, with the nest 10b including a frame-shaped support plate 12 having an upper surface 14, a lower surface 16, a peripheral edge 18, and an inner edge 24 - with the plate defining a hollow interior area 22. The chimneys 34 include a plurality of perimeter chimneys 34a and interior chimneys 34b, with the perimeter chimneys 34a forming a perimeter about the entire arrangement of chimneys 34, while the interior chimneys 34b are surrounded by the perimeter chimneys 34a. The perimeter chimneys 34a may be directly joined to (i.e., formed integrally with) the support plate 12, with a portion of the bottom end of each perimeter chimney 34a joined to the support plate 12. That is, each of perimeter chimneys 34a has a bottom end that is partially joined to the frame-shaped support plate 12 along the inner edge 24 thereof. Conversely, the interior chimneys 34b are positioned within the hollow interior area 22 of the frame-shaped support plate 12 and are not directly joined to the support plate 12, but instead are only joined to perimeter chimneys 34a via reinforcing ribs 48.

A plurality of radial reinforcing ribs 48 connect adjacent chimneys 34 one to each other for rigidifying the nest 10. Each of the ribs 48 is structured as a planar, wall-shaped member that is oriented vertically and orthogonal to the support plate 12, and each rib 48 extends between an adjacent pair of chimneys 34, with the rib 48 being formed integrally with the chimneys 34 as part of a single nest structure. For an embodiment where the chimneys 34 are provided in a plurality of aligned rows and columns, as shown in FIG. 4, each interior chimney 34b will include four (4) ribs 48 extending radially outward therefrom to four (4) adjacent chimneys 34 arranged thereabout, with the ribs 48 arranged in a cross-shaped pattern. Each perimeter chimney 34a will include two or three ribs 48 extending radially outward therefrom to adjacent chimneys 34. As previously described, the ribs 48 are constructed to have a height, H_{Rib}, that is at least a quarter the height of the chimneys, H_{Chimney}, with a bottom edge of the ribs 48 being generally aligned with the bottom end of chimneys 34 and extending upwardly therefrom. According to some embodiments, each of ribs 48 may be constructed to have a height, H_{Rib}, that is equal to the height of the chimneys, H_{Chimney}, such that the ribs 48 extend a full length of the chimneys 34, between the bottom end and the top end thereof.

With the nest 10b structured as described above, the performance and longevity of the nest 10b can be improved as compared to previous nest designs. That is, the increased height of the reinforcing ribs 48 formed between chimneys 34 improves the ability of the nest 10b to resist deflection or deformation when the nest 10b is fully filled with medical containers, while the frame-shaped support plate 12 (with hollow interior area 22) aids in addressing flatness issues that may arise after molding and/or after sterilization of the nest 10b.

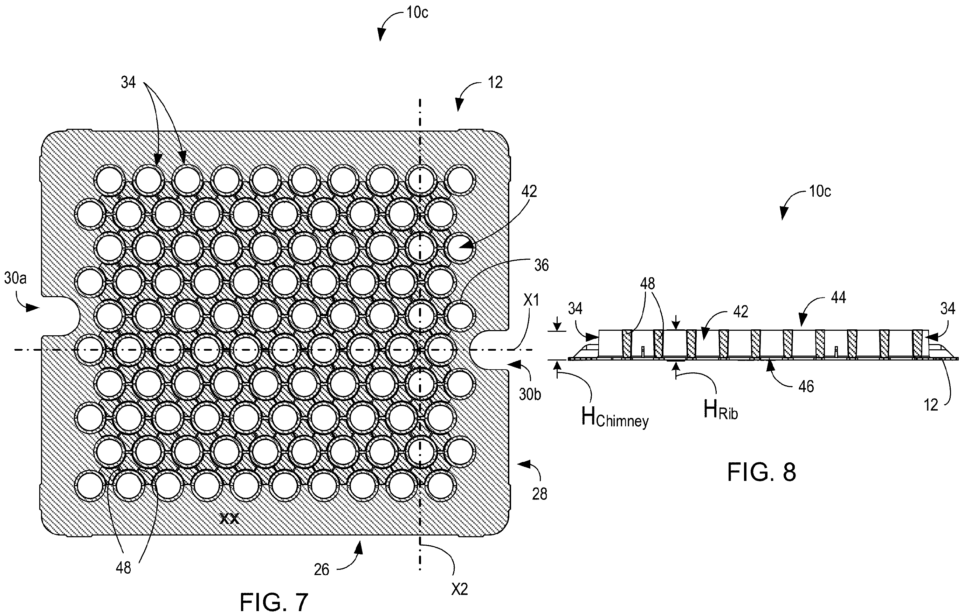

FIGS. 7 and 8 illustrate another example of a nest 10c including optimized reinforcing ribs 48. The nest 10c is identical to the nest 10a of FIGS. 1-3, except that support plate 12 is structured as to exclude the hollow interior area 22 of the previously described embodiment - i.e., the support plate 12 is a rectangular plate with no hollow interior area/region. With the support plate 12 structured as a more continuous plate, each of chimneys 34 is joined to the support plate 12 at a bottom end of the chimneys 34.

The nest 10c includes radial reinforcing ribs 48 connect adjacent chimneys 34 one to each other for rigidifying the nest 10, as previously described in detail. Again, the ribs 48 are constructed to have a height, H_{Rib}, that is at least a quarter the height of the chimneys, H_{Chimney}, with a bottom edge of the ribs 48 being generally aligned with the bottom end of chimneys 34 and extending upwardly therefrom at least to the vertical midpoint of the chimney 34. According to some embodiments, each of ribs 48 may be constructed to have a height, H_{Rib}, that is equal to the height of the chimneys, H_{Chimney}, such that the ribs 48 extend a full length of the chimneys 34, between the bottom end and the top end thereof. With the ribs 48 configured as such, the nest 10c exhibits an increased rigidity that improves the ability of the nest 10c to resist deflection or deformation when the nest 10c is fully filled with medical containers.

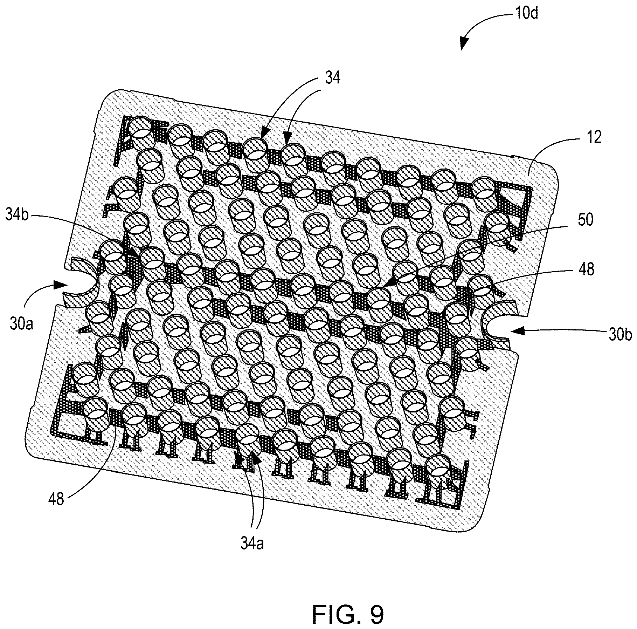

FIG. 9 illustrates another exemplary nest 10d including optimized reinforcing ribs 48. The nest 10d is identical to the nest 10c of FIGS. 7 and 8 regarding the structure of support plate 12 and the arrangement of chimneys 34, but the nest 10d includes a modified arrangement of reinforcing ribs 48. Regarding the arrangement of reinforcing ribs 48 provided in nest 10d, it is recognized that the inclusion of ribs 48 at certain specified locations on nest 10d contributes more to increasing the rigidity/stiffness of the nest 10d than the inclusion of ribs 48 at other locations - such that ribs 48 are not required between all adjacent pairs of chimneys 34. In the illustrated embodiment, ribs 48 are provided between all adjacent perimeter chimneys 34a and between each pair of adjacent chimneys 34 that extend in a line 50 between the first cutout 30a and the second cutout 30b of the nest 10d. Additionally, ribs 48 may be provided between at least some adjacent interior chimneys 34b positioned adjacent/near the longitudinal sides 26 of the support plate 12. As determined by FEA, the inclusion of ribs 48 between chimneys 34 at these locations contributes the most toward increasing rigidity/stiffness of the nest 10d.

The nests of the present disclosure can be made of various plastic materials commonly used for disposable medical packaging, containers, and/or devices. Further, the nests can be made by various forming and molding processes, as are known in the art. For example, the nests of the present disclosure can comprise a thermoplastic material, preferably polypropylene, or polyester, polycarbonate, polyethylene, polyethylene terephthalate, acrylonitrile butadiene styrene, or other injection moldable or formable resin materials, as are known in the art. In some examples, the support plate 12 and chimneys 34 can be integrally formed from a same material, such as from the same thermoplastic material. In some examples, the nests are formed by a single injection molding process, in which the nest is formed by injecting a fluid polymer material between a first tool and a second tool in a single process. In other examples, the nests 10a-10d can be made by three-dimensional printing by processes known in the art.

Beneficially, embodiments of the disclosure thus provide a nest with increased stiffness that reduces deflection of the nest or tray during manufacture, transport, and/or filling. The nest includes reinforcing ribs of increased height that improve the ability of the nest to resist deflection or deformation when the nest is fully filled with medical containers. According to some aspects of the disclosure, the nest includes a frame-shaped plate having a hollow interior area, with a portion of the support plate removed that may contribute to unpredictable curvature and/or deflection (i.e., "flatness issues") of the nest after molding and/or after sterilization thereof. Avoiding deformation of the nest during resulting from the molding process and/or loading of the nest with medical containers can improve accuracy of the filling process by avoiding misalignment between the medical containers contained by the nest and the automated filling machines and also allows for improvements in inserting/removing the medical containers into/out of the nest.

While examples of the support plates are shown in the accompanying figures and described hereinabove in detail, other examples will be apparent to, and readily made by, those skilled in the art without departing from the scope and spirit of the invention. Accordingly, the foregoing description is intended to be illustrative rather than restrictive. The invention described hereinabove is defined by the appended claims and all changes to the invention that fall within the meaning and the range of equivalency of the claims are to be embraced within their scope.

## Claims

1. A nest configured to support medical containers, comprising:
a support plate comprising a first surface, a second surface, and a peripheral edge between the first surface and the second surface, with the support plate configured as a frame-shaped plate having a hollow interior area;
a plurality of chimneys extending outwardly and away from the first surface, each of the plurality of chimneys defining a receptacle configured to receive a medical container therein, with each of the plurality of chimneys having a top end defining an upper opening and a bottom end defining a lower opening, wherein the plurality of chimneys comprise:
an interior chimney group comprising a plurality of interior chimneys, the interior chimney group positioned in the hollowed interior area of the frame-shaped plate; and
a perimeter chimney group surrounding the interior chimney group, the perimeter chimney group comprising a plurality of perimeter chimneys, with each of the plurality of perimeter chimneys at least partially joined to an inner edge of the frame-shaped plate at the bottom end thereof; and
reinforcing ribs extending between the plurality of chimneys, the reinforcing ribs connecting each respective chimney to a plurality of adjacent chimneys.

2. The nest of claim 1, wherein the reinforcing ribs are orthogonal to the support plate and extend up from a bottom end of a respective chimney to a height that is a quarter or greater than a height of the chimney.

3. The nest of claim 2, wherein the height of each of the reinforcing ribs is equal to the height of each of the plurality of chimneys, such that the reinforcing rib extends from the bottom end to the top end of each chimney.

4. The nest of any of claims 1-3, wherein an arrangement of six reinforcing ribs connects each of the plurality of interior chimneys to six adjacent chimneys in a hexagonal arrangement.

5. The nest of any of claims 1-3, wherein an arrangement of four reinforcing ribs connects each of the plurality of interior chimneys to four adjacent chimneys in a cross-shaped arrangement.

6. The nest of any of claims 1-5, further comprising a plurality of plate sections positioned in the hollow interior area and in a planar arrangement with the support plate, each of the plate sections positioned between a grouping of chimneys of the plurality of interior chimneys, wherein a bottom edge of each of the reinforcing ribs joining a respective grouping of chimneys is joined with a respective plate section.

7. The nest of any of claims 1-6, wherein the plurality of chimneys are configured to receive syringes therein from 0.5 to 50 mL in volume.

8. The nest of any of claims 1-7, wherein each of the plurality of chimneys comprises a tubular wall comprising a cylindrical inner surface having a diameter substantially the same as the diameter of the upper and lower openings of the chimney and a cylindrical outer surface opposite the inner surface.

9. The nest of any of claims 1-8, wherein the support plate, the plurality of chimneys, and the reinforcing ribs are integrally formed and are made from a thermoplastic polymer.

10. The nest of any of claims 1-9, wherein, when fully loaded with the medical containers, deflection of the support plate is less than 1.0 mm in a vertical direction.

11. A nest configured to support medical containers, comprising:
a support plate comprising a first surface, a second surface, and a peripheral edge between the first surface and the second surface;
a plurality of chimneys extending outwardly and away from the first surface, each of the plurality of chimneys defining a receptacle configured to receive a medical container therein, with each of the plurality of chimneys having a top end defining an upper opening and a bottom end defining a lower opening; and
reinforcing ribs extending between at least some of the plurality of chimneys, the reinforcing ribs connecting a respective chimney to one or more adjacent chimneys;
wherein the reinforcing ribs are orthogonal to the support plate and extend up from the bottom end of a respective chimney to a height that is a quarter or greater of a height of the chimney.

12. The nest of claim 11, wherein the height of each of the reinforcing ribs is equal to the height of each of the plurality of chimneys, such that the reinforcing rib extends from the bottom end to the top end of each chimney.

13. The nest of claim 11 or claim 12, wherein the plurality of chimneys comprises:
an interior chimney group comprising a plurality of interior chimneys; and
a perimeter chimney group surrounding the interior chimney group, the perimeter chimney group comprising a plurality of perimeter chimneys.

14. The nest of claim 13, wherein the support plate comprises a frame-shaped plate having a hollow interior area, with the interior chimney group positioned in the hollowed interior area of the frame-shaped plate and with each of the plurality of perimeter chimneys at least partially joined to an inner edge of the frame-shaped plate at the bottom end of the chimney.

15. The nest of claim 13, wherein the support plate comprises a pair of cutouts extending inwardly from the peripheral edge thereof, with a first cutout formed on a first transverse side of the support plate and a second cutout formed on a second transverse side of the support plate, and wherein the reinforcing ribs comprise:
a first arrangement of reinforcing ribs connecting each of a plurality of chimneys in a line of chimneys arranged between the first cutout and the second cutout; and
a second arrangement of reinforcing ribs connecting each of the plurality of perimeter chimneys to an adjacent perimeter chimney.

16. The nest of claim 11, wherein an arrangement of reinforcing ribs connects each of the plurality of interior chimneys to chimneys adjacent thereto, the arrangement of reinforcing ribs comprising an arrangement of six reinforcing ribs connecting each of the plurality of interior chimneys to six adjacent chimneys or an arrangement of four reinforcing ribs connecting each of the plurality of interior chimneys to four adjacent chimneys in a cross-shaped arrangement.
